# EUROPÄISCHE PATENTSCHRIFT

(11) **EP 0 820 454 B1**
(45) Veröffentlichungstag und Bekanntmachung des Hinweises auf die Patenterteilung: **24.07.2002**
(21) Anmeldenummer: 96909034.9
(22) Anmeldetag: 03.04.1996
(51) Int. Cl.: C07D 471/14, C07D 471/04, C07D 498/14, C07D 513/14

(54) **ANELLIERTE BETA-CARBOLINE**
ANELLATED BETA-CARBOLINES
BETA-CARBOLINES ANNELEES

(30) Priorität: 12.04.1995 DE 19514524
(43) Veröffentlichungstag der Anmeldung: 28.01.1998
(73) Patentinhaber: SCHERING AKTIENGESELLSCHAFT, 13353 Berlin (DE)
(72) Erfinder: SEIDELMANN, Dieter, D-12159 Berlin (DE); HUTH, Andreas, D-12437 Berlin (DE); OLESEN, Preben, H., DK-2400 Kobenhavn NV (DK); OTTOW, Eckhard, D-12203 Berlin (DE); TURNER, Jonathan, D-13465 Berlin (DE); HILLMANN, Margrit, D-13437 Berlin (DE); COLE, Belinda, D-10625 Berlin (DE)
(86) Internationale Anmeldenummer: DE9600632
(87) Internationale Veröffentlichungsnummer: WO9632392

(56) Entgegenhaltungen:
- DE-A- 4 130 933
- CHEMICAL ABSTRACTS, vol. 96, no. 3, 1982 Columbus, Ohio, US; abstract no. 15916j, PERIN -ROUSSEL ET AL.: "Apparent stimulation of dibenzo[a,e]fluoranthene in vitro metabolism in the presence of norharman" Seite 100; XP002006760 & CHEM.-BIOL. INTERACT., Bd. 37, 1981, Seiten 109-122,
- CHEMICAL ABSTRACTS, vol. 114, no. 3, 1991 Columbus, Ohio, US; abstract no. 23869b, AGARWAL ET AL.: "Antiparasitic agents. Part X. Synthesis of 2,7-disubstituted-1,6-dihydropyrido[3,4-b] imidazo[4,5-e]indoles as anthelmintic agents" Seite 698; XP002006761 & INDIAN J. CHEM., SECT B, Bd. 29B, Nr. 9, 1990, Seiten 843-47,

## Beschreibung

Die Erfindung betrifft anellierte β-Carboline, deren Herstellung und Verwendung in Arzneimitteln.

Aus zahlreichen Publikationen ist bekannt, daß β-Carboline Affinität an die Benzodiazepin-Rezeptoren besitzen, obwohl sie sich strukturell von Benzodiazepinen unterscheiden und daß sie auf Grund der Affinität an die BDZ-Rezeptoren als Psychopharmaka verwendet werden. β-Carboline können auf die von BDZ-Rezeptoren bekannten Eigenschaften antagonistisch, agonistisch oder invers agonistisch wirken.

Es wurde nun gefunden, daß die erfindungsgemäßen Verbindungen eine sehr gute Affinität an die Benzodiazepin-Rezeptoren besitzen und auf die von Benzodiaze-pinen bekannten Eigenschaften spezifisch invers agonistisch wirken. Die Verbindungen besitzen anxiolytische, antiamnestische und nootrope Aktivitäten und verbessern das Lernen und die Aufmerksamkeit. Auf Grund ihres Wirkprofils eignen sich die erfindungsgemäßen Verbindungen zur Herstellung von Arzneimitteln zur Behandlung geriatrischer Beschwerden sowie zur Abschwächung cognitiver Defizite und zur Vigilanzsteigerung, ohne daß gravierende Nebenwirkungen auftreten.

Die Erfindung betrifft die Verbindungen der Formel I, deren optische Isomere, Tautomere und physiologisch verträglichen Salze 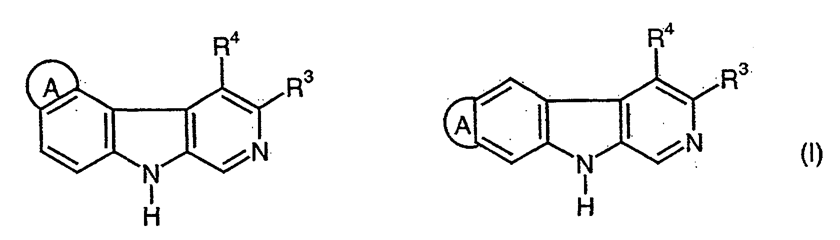 worin
R³ -CO-R¹,
R⁴ Wasserstoff, C₁₋₆-Alkyl, C₁₋₄-Alkoxy-C₁₋₂-alkyl,
A einen 5-6 gliedrigen ungesättigten Ring darstellt, in dem 1 - 2 C-Atome durch N, O und/oder S ersetzt sein können und der mit R⁵ und R⁶ substituiert sein kann, und
R⁵ und R⁶ gleich oder verschieden sind und Wasserstoff, C₁₋₆-Alkyl, C₁₋₆-Alkoxy, Hydroxy, das mit leicht abspaltbaren Etherresten, C₁₋₆-Alkanoyl, Benzoyl, zu 1,3-Dioxan, zu 1,3-Dioxolan, zu 2-Phenyl-1,3-dioxan oder zu 2,2-Dimethyl-1,3-dioxolan funktionell abgewandelt sein kann, NR⁷R⁸, COR, C₁₋₆-Alkyl, das gegebenenfalls mit leicht abspaltbaren Etherresten, C₁₋₆-Alkanoyl, Benzoyl, zu 1,3-Dioxan, zu 1,3-Dioxolan, zu 2-Phenyl-1,3-dioxan oder zu 2,2-Dimethyl-1,3-dioxolan funktionell abgewandeltem Hydroxy, C₁₋₄-Alkoxy oder Halogenen substituiert ist, einen C₆₋₁₂-Aryl oder einen 5-6-gliedrigen Hetarylrest, der ein- bis drei N-, O- und/oder S-Atome enthält, bedeuten und der Aryl- und Hetarylrest ein- oder mehrfach mit C₁₋₄-Alkyl, C₁₋₄-Alkoxy, Halogen oder CF₃ substituiert sein kann, oder
R⁵ und R⁶ gemeinsam eine -(CH₂)ₙ-Gruppe bedeuten und
R¹ und R Hydroxy, C₁₋₆-Alkoxy oder NR¹⁰R¹¹,
n 3 oder 4,
R⁷ und R⁸ jeweils Wasserstoff, C₁₋₆-Alkyl, Acyl oder Phenyl, das ein- oder mehrfach mit
C₁₋₄-Alkyl, C₁₋₄-Alkoxy, Halogen oder CF₃ substituiert sein kann,
R¹⁰ und R¹¹ jeweils Wasserstoff, C₁₋₆-Alkyl, C₃₋₇-Cycloalkyl oder einen C₆₋₁₂-Aryl- oder einen 5-6-gliedrigen Hetarylrest, der ein- bis drei N-, O- und/oder S-Atome enthält,
bedeuten und der Aryl- und Hetarylest ein- oder mehrfach mit C₁₋₄-Alkyl, C₁₋₄-Alkoxy, Halogen oder CF₃ substituiert sein kann.

Alkyl beinhaltet jeweils sowohl gerad- als auch verzweigt-kettige Reste wie beispielsweise Methyl, Ethyl, Propyl, Isopropyl, Butyl, Isobutyl, sek. Butyl, tert. Butyl, Pentyl, Isopentyl und Hexyl.

Als Arylrest R⁵, R⁶, R¹⁰ oder R¹¹ seien beispielsweise Phenyl, Biphenyl und α- oder β-Naphthyl genannt, die gegebenenfalls 1-3-fach substituiert sind.

Bedeutet R⁵, R⁶, R¹⁰ oder R¹¹ einen Hetarylrest, so sind Sechsringheteroaromaten mit bis zu 3 Stickstoffatomen und Fünfringheteroaromaten mit ein bis zwei Sauerstoff-, Schwefel- und/oder Stickstoffatomen gemeint, wie beispielsweise Triazin, Pyridin, Pyrimidin, Pyrazin, Pyridazin, Furan, Thiophen, Pyrrol, Imidazol, Thiazol, 1,2,4-Oxadiazolyl, 1,3,4-Oxadiazolyl, die jeweils 1-3-fach substituiert sein können.

Unter Halogen ist jeweils Fluor, Chlor, Brom und Jod zu verstehen. Cycloalkyl steht jeweils für Cyclopropyl, Cyclobutyl, Cyclopentyl, Cyclohexyl, Cycloheptyl.

Der Alkylrest R⁵, R⁶ kann 1-3-fach substituiert oder auch perhalogeniert vorliegen.

Die Hydroxygruppen können funktionell abgewandelt sein, beispielsweise durch Veretherung oder Veresterung. Als Ether- und Acylreste kommen die dem Fachmann bekannten Reste in Betracht. Bevorzugt sind leicht abspaltbare Etherreste, wie beispielsweise der Tetrahydropyranyl-, Tetrahydrofuranyl-, tert.-Butyldimethylsilyl-, tert.-Butyldiphenylsilyl-, Tribenzylsilylrest. Als Acylreste kommen zum Beispiel C₁₋₆-Alkanoyle wie Acetyl, Propionyl, Butyryl und Benzoyl in Frage.

Sind mehrere Hydroxygruppen vorhanden, so können cyclische Acetale oder Ketale vorliegen, wie 1,3-Dioxan- oder 1,3-Dioxolanreste wie 2-Phenyl-1,3-dioxan, 2,2-Dimethyl-1,3-dioxolan, die beispielsweise durch Umsetzung mit Aceton, einem Enolether, 1,1-Dihalogenalkan oder Acetondimethylketal dargestellt werden.

Die Acylgruppe R⁷ oder R⁸ beinhaltet aromatische und aliphatische Acylgruppen wie Benzoyl und ein- bis dreifach substituierte Benzoyle sowie geradkettige oder verzweigte Alkanoyle mit bis zu 6 Kohlenstoffatomen.

Beinhaltet A einen heteroaromatischen Fünfring, so kann dieser die folgenden Gruppierungen besitzen:

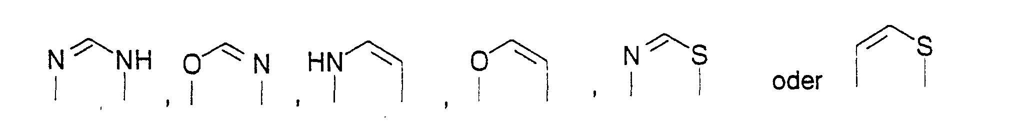

Als heteroaromatischer Sechsring seien beispielsweise die folgenden Gruppierungen genannt:

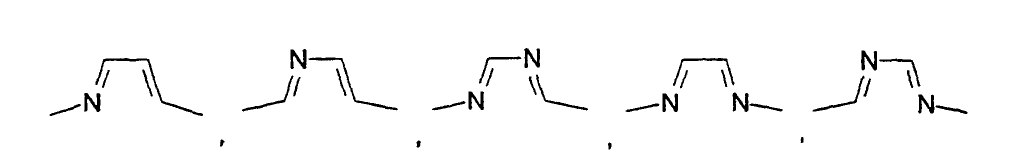

Die Substituenten R⁵ und R⁶ können jeweils in beliebiger Position am Rest A oder dessen tautomeren oder isomeren Formen stehen. Als bevorzugte Ausfuhrungsformen von R³ ist -COR¹ und von R¹ und R Hydroxy und C₁₋₆-Alkoxy zu betrachten.

Ist eine basische Funktion vorhanden, so leiten sich die physiologisch verträglichen Salze von anorganischen und organischen Säuren ab. Geeignet sind anorgische Säuren wie beispielsweise Chlorwasserstoffsäure, Bromwasserstoffsäure, Schwefelsäure oder organische Säuren wie beispielsweise aliphatische oder aromatische Mono- oder Dicarbonsäuren wie Ameisensäure, Essigsäure, Maleinsäure, Fumarsäure, Succinsäure, Milchsäure, Weinsäure, Zitronensäure, Oxalsäure, Glyoxylsäure oder Sulfonsäuren, beispielsweise C₁₋₄-Alkansulfonsäuren wie Methansulfonsäure oder gegebenenfalls durch Halogen oder C₁₋₄-Alkyl substituierte Benzolsulfonsäuren wie p-Toluolsulfonsäure.

Ist eine saure Funktion vorhanden, so sind als Salze die physiologisch verträglichen Salze organischer Basen geeignet wie beispielsweise die gut löslichen Alkali- und Erdalkalisalze sowie N-Methyl-glukamin, Dimethyl-glukamin, Ethyl-glukamin, Lysin, 1,6-Hexadiamin, Ethanolamin, Glukosamin, Sarkosin, Serinol, Tris-hydroxy-methyl-amino-methan, Aminopropandiol, Sovak-Base, 1-Amino-2,3,4-butantriol.

Die Verbindungen der Formel I sowie deren Salze sind auf Grund ihrer Affinität zu Benzodiazepin-Rezeptoren als Arzneimittel verwendbar. Sie besitzen unterschiedliche intrinsische Wirksamkeit (.d.h. agonistische, antagonistische und/oder invers agonistische Wirksamkeit) an verschiedenen Isoformen des GABA-Benzodiazepin-Rezeptors.

Zur Verwendung der erfindungsgemäßen Verbindungen als Arzneimittel werden diese in Form eines pharmazeutischen Präparats gebracht, das neben dem Wirksstoff für die enterale oder parenterale Applikation geeignete pharmazeutische, organische oder anorganische inerte Trägermaterialien, wie zum Beispiel Wasser, Gelatine, Gummi arabicum, Milchzucker, Stärke, Magnesiumstearat, Talk, pflanzliche Öle, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüber hinaus Hilfsstoffe wie Konservierungs-, Stabilisierungs-, Netzmittel oder Emulgatoren, Salze zur Veränderung des osmotischen Drucks oder Puffer.

Für die parenterale Anwendung sind insbesondere Injektionslösungen oder Suspensionen, insbesondere wässrige Lösungen der aktiven Verbindungen in polyhydroxyethoxyliertem Rizinusöl, geeignet.

Als Trägersysteme können auch grenzflächenaktive Hilfsstoffe wie Salze der Gallensäuren oder tierische oder pflanzliche Phospholipide, aber auch Mischungen davon sowie Liposome oder deren Bestandteile verwendet werden.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke geeignet. Die Anwendung kann auch in flüssiger Form erfolgen, wie zum Beispiel als Saft, dem gegebenenfalls ein Süßstoff beigefügt wird. Die erfindungsgemäßen Verbindungen werden in einer Dosiseinheit von 0,05 bis 100 mg aktiver Substanz in einem physiologisch verträglichen Träger eingebracht.

Die Dosierung der Wirkstoffe kann je nach Verabfolgungsweg, Alter und Gewicht des Patienten, Art und Schwere der zu behandelnden Erkrankung und ählichen Faktoren variieren. Die tägliche Dosis beträgt 0,1 - 300 mg, vorzugsweise 0,1 - 30 mg, wobei die Dosis als einmal zu verabreichende Einzeldosis oder unterteilt in 2 oder mehrere Tagesdosen gegeben werden kann.

Die Herstellung der erfindungsgemäßen Verbindungen erfolgt nach an sich bekannten Methoden. Beispielsweise gelangt man zu Verbindungen der Formel I dadurch, daß man
a) eine Verbindung der Formel II 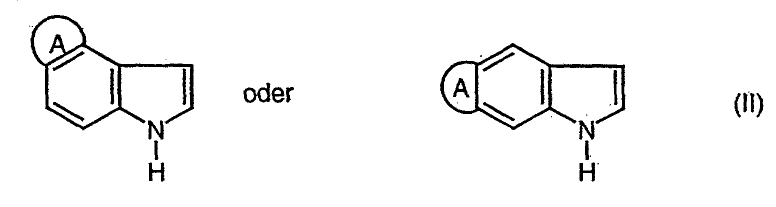 mit einem 2-Azadien der Formel III 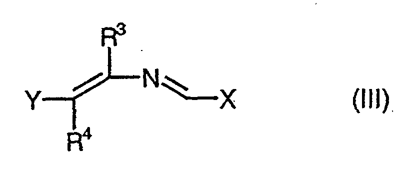 worin R³, R⁴ und A die obige Bedeutung haben, und X und Y Fluchtgruppen darstellen, in Gegenwart von Säuren umsetzt oder
b) eine Verbindung der Formel IV 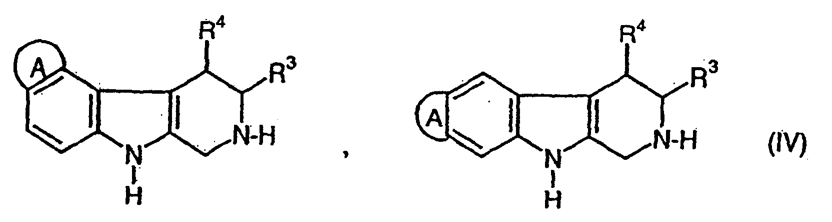 worin R³, R⁴ und A die obige Bedeutung haben, aromatisiert oder
c) eine Verbindung der Formel V 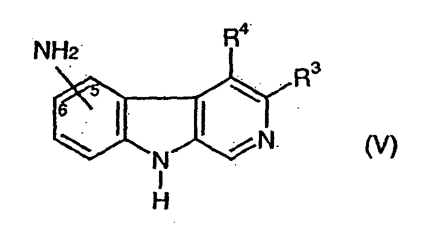 worin R³ und R⁴ die obige Bedeutung haben, mit einem α,β-ungesättigtem Aldehyd umsetzt zu einem ankondensierten Pyridin oder mit einem primären Amin H₂N-CH₂-R⁵ umsetzt zu einem ankondensierten Imidazol oder die mit Nitriten erhaltenen Diazoniumsalze mit Acetessigsäurederivaten umsetzt zu einem Ethylidenhydrazinderivat und dieses zum Pyrrol cyclisiert oder mit Thiocyanat- oder Isothiocyanatderivaten umsetzt zu einem ankondensierten Thiazol oder
d) eine Verbindung der Formel VI 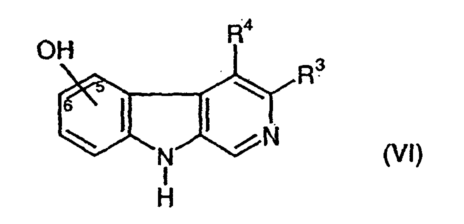 worin R³ und R⁴ die obige Bedeutung haben, mit einem primären Amin H₂N-CH₂-R⁵ zu einem ankondensierten Oxazol umsetzt oder mit einem vicinalen primären Diamin 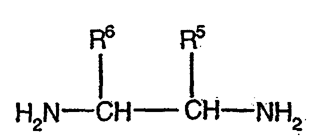 zu einem ankondensierten Pyrazin umsetzt oder
e) ein Nitriloxid der Formel VII 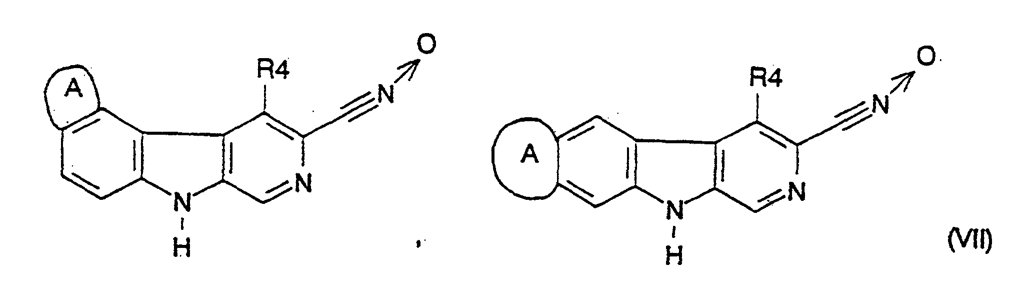 mit ≡―R² zu einem Isoxazolderivat cyclisiert,
und gewünschtenfalls anschließend eine Estergruppe verseift oder umestert, eine Carboxylgruppe verestert, eine Aminogruppe alkyliert oder acyliert, eine funktionell abgewandelte Hydroxygruppe freisetzt, die Isomeren trennt oder die physiologisch verträglichen Salze bildet.

Die erfindungsgemäße Umsetzung von Verbindungen der Formel II mit 2-Azadienen der Formel III zu den Verbindungen der Formel I nach dem Verfahren a) erfolgt nach EP-A-110813 in Gegenwart von Säuren bei Temperaturen von 0 bis 150 °C. Die Fluchtgruppen X und Y können gleich oder verschieden sein; insbesondere geeignet sind C₁₋₃-Dialkylamine, wie Dimethyl-, Diethyl- und Diisopropylamin, und cyclische Amine, wie Pyrrolidin.

Die Umsetzung wird beispielsweise so ausgeführt, daß das Indolderivat und das Azadien in einer organischen Säure, wie zum Beispiel Ameisensäure, Essigsäure, Propionsäure oder Trifluoressigsäure, zunächst bei Raumtemperatur gerührt und dann bis zur Siedetemperatur des Reaktionsgemisches erhitzt wird.

Die Säure kann gleichzeitig als Reaktionsmittel und auch als Lösungsmittel dienen. Es können aber auch Lösungsmittel wie zum Beispiel Alkohole, Ether, Ketone, Ester, wie Ethylacetat, Kohlenwasserstoffe, wie Toluol, oder Halogenkohlenwasserstoffe, wie Tetrachlorkohlenstoff, zugesetzt werden.

Die Menge der Säure kann in weiten Grenzen variiert werden, sie wird jedoch im Überschuß angewendet. Vorzugsweise wird ein 3 - 10 facher Säureüberschuß, bezogen auf das Azadien, gewählt.

Die Mölverhältnisse von Indol und Azadien sind für den Erfolg der Umsetzung nicht kritisch. Im allgemeinen wird man etwa gleiche molare Mengen der Reationspartner einsetzen, wobei Mengenverhältnisse von 1 Mol Anilin und 1-3 Mol Azadien bevorzugt sind. Die erfindungsgemäße Umsetzung kann grundsätzlich auch in den oben angegebenen Lösungsmitteln mit katalytischen Mengen von Mineralsäuren, wie Schwefelsäure, Salzsäure, Perchlorsäure oder organischen Säuren wie p-Toluolsulfonsäure und Trifluoressigsäure, durchgeführt werden.

Zur Aromatisierung der Verbindungen der Formel IV sind die von den β-Carbolinen bekannten Verfahren geeignet wie beispielsweise die Dehydrierung mit tert. Butylhypochlorit (EP-A-190 987) oder mit Trichlorisocyanursäure (WO 94/12 498).

Die Ankondensation des ungesättigten Ringes A nach Verfahrensvariante c) und d) erfolgt in Abhängigkeit der Stellung der Amino- oder Hydroxy-Gruppe in 5,6- oder 6,7-Stellung des β-Carbolins, vorzugsweise in 5,6-Stellung. Eventuell anfallende Isomerengemische werden in üblicher Weise durch fraktionnierte Kristallisation oder Chromatographie getrennt.

Wird ein Pyridinring synthetisiert, so kann dies nach der Synthese von Skraup [G. Alunni-Bistocchi et al. J. Chem. Soc. Perkin Trans. 1, 2935 (1992)] erfolgen, indem man beispielsweise einen α, β-ungesättigten Aldehyd, der intermediär erzeugt werden kann, an das Amin addiert und anschließend unter Einfluß von Säuren cyclisiert und mit einem Oxidationsmittel wie Arsenpentoxid, EisenIIIoxid oder Pikrinsäure aromatisiert. Die Reaktion wird bei Temperaturen von Raumtemperatur bis 150 °C in inerten Lösungsmitteln wie Toluol, Xylol vorgenommen.

Zur Herstellung eines Imidazols kondensiert man beispielsweise das entsprechende Amino-β-carbolinderivat mit einem primären Amin R⁵-CH₂-NH₂ in Gegenwart eines Oxidationsmittels wie MnO₂ bei Raumtemperatur oder erhöhter Temperatur in inerten Lösungsmitteln wie Dichlormethan, Dichlorethan oder Ethylenglykoldimethylether.

Das Pyrrolocarbolin kann z.B. aus dem Ethylenhydrazino-β-carbolinderivat hergestellt werden, indem man dieses in einem inerten Lösungsmittel wie Kohlenwasserstoffen z.B. Toluol, Xylol, Benzol, in Gegenwart von organischen oder anorganischen Säuren oder von Polyphosphorsäureestern erhitzt.

Die Darstellung der Ethylenhydrazino-Ausgangsverbindungen kann mit Hilfe der Sandmeyer-Reaktion erfolgen, indem man z.B. die aus den Aminoverbindungen mit Nitriten intermediär gebildeten Diazoniumsalze mit Alkalisalzen des Acetessigsäureesters in protischen Lösungsmitteln wie Wasser oder Alkoholen bei Temperaturen von 0°C bis Raumtemperatur umsetzt.

Thiazolo-carboline können beispielsweise durch Umsetzung der Verbindungen der Formel V mit Thiocyanat- oder Isothiocyanatverbindungen dargestellt werden. Die Umsetzung erfolgt zweckmäßigerweise in einem inerten Lösungsmittel in Gegenwart einer organischen oder anorganischen Säure, wobei falls eine organische Säure eingesetzt wird, diese als Lösungsmittel dienen kann. Zur Cyclisierung wird im allgemeinen ein Oxidationsmittel wie zum Beispiel Brom hinzugefügt. Ausgehend von Hydroxy-β-carbolinen erhält man in Analogie zu der vorne beschriebenen Skraup-Synthese durch Umsetzung eines primären Amins in Gegenwart eines Oxidationsmittels wie MnO₂ bei Raumtemperatur oder erhöhter Temperatur in einem inerten Lösungsmittel Oxazolo-carboline. Wird bei der Umsetzung statt eines primären Amins eine vicinale primäre Diaminoverbindung eingesetzt, so erhält man die entsprechenden Pyrazinderivate, deren Isomerengemische in üblicher Weise wie chromatographisch oder durch fraktionnierte Kristallisation getrennt werden können.

Die Umsetzung der Nitriloxide der Formel VII mit den Acetylenderivaten kann beispielsweise nach den von K.G.B. Torsell beschriebenen Methoden erfolgen (K.G.B. Torsell, Nitrile Oxides, Nitrones and Nitronates in Organic Synthesis, 1988 VCH Verlagsgesellschaft mbH). Hierbei wird in der Regel zuerst das Nitriloxid erzeugt, welches dann ohne Isolierung mit einem Acetylenderivat umgesetzt wird.

Die Molverhältnisse von Nitriloxid und Acetylen können in Grenzen variieren, Im allgemeinen wird man etwa gleiche molare Mengen der Reaktionspartner einsetzen, doch kann es oft auch günstig sein, das Acetylenderivat in einem größeren Umfang einzusetzen. Die Umsetzung wird bei Temperaturen von - 78 °C bis 150 °C, vorzugsweise von - 20 °C bis 50 °C, in einem aprotischen Lösungsmittel ausgeführt.

Als Lösungsmittel sind beispielsweise aliphatische und cyclische Ether wie Diethylether, Tetrahydrofuran, Dioxan, halogenierte Kohlenwasserstoffe wie Dichlorethan, Methylenchlorid, Chloroform, Kohlenwasserstoffe wie Hexan, Pentan und Dimethylformamid, Dimethylsulfoxid, geeignet.

Sind die Ausgangsverbindungen gasförmig wie beispielsweise Acetylen, so ist es vorteilhaft, die entsprechenden flüssigen Verbindungen, die eine anschließend leicht abspaltbare Gruppe haben, in die Reaktion einzusetzen. Als leicht abspaltbare Gruppe ist beispielsweise die Trialkylsilylgruppe geeignet. Die Abspaltung erfolgt vor Aufarbeitung des Reaktionsgemisches nach den bekannten Methoden wie beispielsweise durch Zusatz von Basen bei Zimmertemperatur. Geeignete Basen sind zum Beispiel Alkalihydroxide und - alkoholate wie Natrium- oder Kalium-hydroxid, -methylat oder -ethylat oder Fluoride wie Cäsiumfluorid oder Tetra-n-butyl-ammonium-fluorid.

Gegebenenfalls können in die Reaktion in 9-Stellung geschützte β-Carbolin-Derivate eingesetzt werden. Die Schutzgruppe wird in üblicher Weise bei der Aufarbeitung des Reaktionsgemisches oder anschließend durch Behandlung mit Basen oder Säuren je nach Art der Schutzgruppe abgespalten werden.

Die Herstellung der Nitriloxide erfolgt beispielsweise durch Umsetzung von β-Carbolin-3-carbaldehyden zu den entsprechenden Oximen, die beispielsweise mit N-Halogen-Succinimid, tert.Butoxychlorit oder Na-Halogen-Succinimid, tert. Butoxychlorit oder Nahypochlorit in aprotischen Lösungsmitteln in Hydroxamsäurehalogenide überführt werden Ikönnen. Mit Basen wie Na- oder K-alkoholaten, Trialkylaminen, Hünig Base, DBU oder Diazabicyclooctan wird aus den Hydroxamsäurehalogeniden Halogenwasserstoff abgespalten und man erhält die Nitriloxide, die ohne Isolierung der Cycloaddition unterworfen werden (R. Annunziata et al., J. Chem. Soc. 1987, 529).

Die Herstellung der β-Carbolin-3-carbaldehyde kann beispielsweise nach den in EP-305 322 beschriebenen Verfahren aus den β-Carbolin-3-carbonsäurealkylestern erfolgen.

Die Umsetzung mit α-Halogenketonen nach Verfahren f) erfolgt nach den in The Chemistry of Heterocyclic Compounds Vol. 34 Part 1, Seite 180 ff (1979) beschriebenen Methoden. Beispielsweise wird das Thioamid in Lösung oder in Suspension bei Temperaturen bis zur Siedetemperatur des Reaktionsgemisches mit dem α-Halogenketon, insbesondere dem Chlor- oder Bromketon umgesetzt. Als inerte Lösungsmittel sind Alkohole, cyclische und acyclische Ether, Ester, Kohlenwasserstoffe und halogenierte Kohlenwasserstoffe geeignet.

Die Darstellung der 3-Tetrazolyl-β-carboline kann beispielsweise nach den in EP-A-54507 beschriebenen Verfahren mit HN₃ erfolgen oder nach den in E.W. Thomas, Synthesis (1993) S. 767, P. Ornstein et. al. J. Med. Chem 36 2046, (1993) beschriebenen Methoden.

Die Hydrolyse einer Estergruppe kann in üblicher Weise sauer oder alkalisch erfolgen, beispielsweise mit wässrigen Alkali- oder Erdalkalilösungen, gegebenenfalls unter Zusatz von organischen Lösungsmitteln wie Alkoholen bei Temperaturen von Raumtemperatur bis 150 °C oder nach den in EP-A-161 574 beschriebenen Verfahren.

Wird eine Umesterung gewünscht, so kann man die in EP-A-237 467 beschriebenen Methoden anwenden, indem man mit Alkalialkoholaten oder dem entsprechenden Alkohol, gegebenenfalls unter Zugabe von Titan-tetra-isopropylat als Katalysator bei erhöhter Temperatur umestert. Die Einführung der tert. Butylestergruppe erfolgt z. B. durch Umsetzung der Carbonsäure mit tert. Butoxy-bis-dimethyl-aminomethan.

Die Veresterung der Carbonsäure geschieht in an sich bekannter Weise beispielsweise mit dem entsprechenden Alkohol in Säure oder in Gegenwart eines aktivierten Säurederivats. Als aktivierte Säurederivate kommen zum Beispiel Säurechlorid, -imidazolid oder -anhydrid in Frage.

Wird eine Alkylierung der Aminogruppe gewünscht, so kann nach üblichen Methoden beispielsweise mit Alkylhalogeniden alkyliert werden. Die Acylierung der Aminogruppe erfolgt nach den bekannten Methoden. Beispielsweise wird in wässrigem Milieu in Gegenwart einer Base mit den entsprechenden Säureanhydriden oder Säurehalogeniden umgesetzt.

Die Freisetzung der funktionell abgewandelten Hydroxygruppen erfolgt nach den dem Fachmann bekannten Methoden. Beispielsweise wird die Abspaltung von Etherschutzgruppen in einer wässrigen Lösung einer organischen Säure, wie zum Beispiel Ameisensäure, Essigsäure, Propionsäure, Trifluoressigsäure, Zitronensäuren u.a. oder in einer wässrigen Lösung einer anorganischen Säure, wie zum Beispiel Salzsäure, oder unter Verwendung von Lewis-Säuren wie Bortrifluoridetherat vorgenommen.

Silylschutzgruppen können beispielsweise mit Fluoriden wie Tetrabutylammoniumfluorid oder Cäsiumfluorid entfernt werden.

Die Verseifung von Acylgruppen wird nach den dem Fachmann bekannten Methoden durchgeführt, wie beispielsweise mit basischen Katalysatoren wie zum Beispiel mit Alkali- oder Erdalkali-carbonaten oder -hydroxiden in einem Alkohol oder der wässrigen Lösung eines Alkohols.

Die Verbindungen der Formel I können auf an sich bekannte Weise aus dem Reaktionsgemisch isoliert und gereinigt werden. Säureadditionssalze können in üblicher Weise in die freien Basen überführt werden und diese gewünschtenfalls in bekannter Weise in physiologisch verträgliche Säureadditonssalze, beispielsweise indem man die Lösung mit einer konzentrierten Lösung der gewünschten Säure versetzt.

Falls die Verbindungen der Formel I ein Chiralitätszentrum enthalten, können die optisch aktiven Verbindungen ausgehend von optisch aktiven Ausgangsverbindungen oder in an sich bekannter Weise aus den Racematen erhalten werden. Die Enantiomerentrennung kann in üblicher Weise beispielsweise durch Chromatographie über optisch akive Trägermaterialien, durch Umsetzung mit optisch aktiven Säuren und anschließende fraktionierte Kristallisation erfolgen.

Zur Bildung der physiologisch verträglichen Säureadditionssalze wird eine Verbindung der Formel I beispielsweise in wenig Alkohol gelöst und mit einer konzentrierten Lösung der gewünschten Säure versetzt.

Soweit die Herstellung der Ausgangsverbindungen nicht beschrieben wird, sind diese bekannt oder analog zu bekannten Verbindungen oder hier beschriebenen Verfahren herstellbar.

Beispielsweise ist die Herstellung der 3-Carbonsäureester der Formel VI in EP-A-130 140 beschrieben und die Herstellung der Verbindungen der Formel V in EP-A-54 507.

Die Affinität an die Benzodiazepin-Rezeptoren wird durch Untersuchung der Fähigkeit von Prüfsubstanzen radioaktiv markierte Benzodiazepine vom Benzodiazepin-Rezeptor zu verdrängen, bestimmt. Zur Untersuchung der anxiolytischen Wirkung werden die Verbindungen im 4-Plattentest nach der Methode von Boissier et al. Eur. J. Pharmacol. 4, 145-150 (1968) getestet.

Die antiamnestische Wirksamkeit kann nach der Methode von BJ. Cole et al Psychopharmacology (1993) 111:465-471 getestet werden (DMTP-Test) und die Aufmerksamkeit kann nach der Methode von J.L. Muir et al. Exp. Brain Res (1992) 89:611-622 getestet werden (9-Hole Box).

So zeigt beispielsweise Isopropyl-11-methoxymethyl-3-methyl-pyrazino[2,3-g]-β-carbolin-10-carboxylat in "Behavioural tests of learning and memory" (z.B. nach Cole et al. 1994, Psychopharmacol. 116, 135-142) in Ratten in Dosen von 10 mg/kg i.p. eine Verbesserung der cognitiven Leistung.

Die nachfolgenden Beispiele sollen das erfindungsgemäße Verfahren erläutern:

### Beispiel 1

### Isopropyl-11-ethyl-3-methyl-pyrazino[2,3-g]-β-carbolin-10-carboxylat und Isopropyl-11-ethyl-2-methyl-pyrazino[2,3-g]-β-carbolin-10-carboxylat

20 g Isopropyl-4-ethyl-6-hydroxy-β-carbolin-3-carboxylat werden in 800 ml Ethylenglycoldimethylether (DME) und 7,2 ml 1,2-Diaminopropan unter Einleitung von Stickstoff bei Raumtemperatur gelöst. Zu der Lösung werden innerhalb von 30 Minuten unter Rühren 175 g Mangan(IV)-oxid portionsweise eigetragen so, daß die Reaktionstemperatur nicht über 28°C ansteigt. Nach beendeter Zugabe des Mangan(IV)-oxids werden weitere 2,9 ml 1,2-Diaminopropan zugegeben. Das Reaktionsgemisch wird unter Stickstoffatmosphäre über Nacht gerührt. Das Reaktionsgemisch wird über Kieselgur filtriert und der Filter-rückstand fünfmal mit je 100 ml DME nachgewaschen. Die vereinigten Filtrate werden fast bis zur Trockene eingeengt und die ausgefallenen Kristalle isoliert. Das erhaltene Roh-kristallisat wird dreimal aus Methanol umkristallisiert.

Man erhält 9,5 g Isopropyl-11-ethyl-3-methyl-pyrazino[2,3-g]-β-carbolin-10-carboxylat vom Schmelzpunkt 236,5-237,5 °C

Die vereinigten Mutterlaugen werden bis zur Trockene eingeengt und anschließend mit Isopropylacetat ausgekocht. Der ungelöste Rückstand wird abfiltriert und viermal aus Methanol umkristallisiert.

Man erhält 265 mg Isopropyl-11-ethyl-2-methyl-pyrazino[2,3-g]-β-carbolin-10-carboxylat vom Schmelzpunkt 215-216 °C

In analoger Weise werden hergestellt:
Isopropyl-11-methoxymethyl-3-ethyl-pyrazino[2,3-g]-β-carbolin-10-carboxylat
   Schmelzpunkt 202-203 °C
Isopropyl-11-methyl-3-ethyl-pyrazino[2,3-g]-β-carbolin-10-carboxylat
   Schmelzpunkt 204-206 °C
Isopropyl-3,11-diethyl-pyrazino[2,3-g]-β-carbolin-10-carboxylat
   Schmelzpunkt 188-190 °C
Isopropyl-11-methoxymethyl-pyrazino[2,3-g]-β-carbolin-10-carboxylat
   Schmelzpunkt 238 °C (Zers.)
Isopropyl-11-methoxymethyl-2,3,4,5-tetrahydroquinoxalino[2,3-g]-β-carbolin-12-carboxylat
   Schmelzpunkt 224-225 °C (Zers.)
Isopropyl-11-methoxymethyl-3-phenyl-pyrazino[2,3-g]-β-carbolin-10-carboxylat
   Schmelzpunkt 262-263 °C
Isopropyl-11-ethyl-3-phenyl-pyrazino[2,3-g]-β-carbolin-10-carboxylat
   Schmelzpunkt 235-236 °C
Isopropyl-11-methoxymethyl-3-methyl-pyrazino[2,3-g]-β-carbolin-10-carboxylat
   Schmelzpunkt 195-197 °C
Isopropyl-3,11-dimethyl-pyrazino[2,3-g]-β-carbolin-10-carboxylat
   Schmelzpunkt 155-160 °C
Isopropyl-11-methoxymethyl-2,3-dimethyl-pyrazino[2,3-g]-β-carbolin-10-carboxylat
   Schmelzpunkt 244-245 °C
Isopropyl-11-ethyl-2,3-dimethyl-pyrazino[2,3-g]-β-carbolin-10-carboxylat
   Schmelzpunkt 233-236 °C
Isopropyl-11-methyl-2,3-dimethyl-pyrazino[2,3-g]-β-carbolin-10-carboxylat
   Schmelzpunkt 305 °C (Zers.)
Isopropyl-11-methoxymethyl-3-propyl-pyrazino[2,3-g]-β-carbolin-10-carboxylat
   Schmelzpunkt 172-173 °C
Isopropyl-11-ethyl-3-propyl-pyrazino[2,3-g]-β-carbolin-10-carboxylat
   Schmelzpunkt 184-186 °C
Isopropyl-11ethyl-3-methoxymethyl-pyrazino[2,3-g]-β-carbolin-10-carboxylat
   Schmelzpunkt 198-199 °C
Isopropyl-3,11-bismethoxymethyl-pyrazino[2,3-g]-β-carbolin-10-carboxylat
   Schmelzpunkt 193-194 °C

### Beispiel 2

### 7H-Benzo[e]pyrido[3,4]-indol-10-carbonsäureethylester

Analog dem Verfahren im Beispiel 1 von EP-110 813 wird aus 3H-Benzo[e]indol und 3-Dimethylamino-2-(dimethylaminomethylenamino)-acrylsäure-ethylester (Azadien 1) die Titelverbindung vom Schmelzpunkt 278 - 280 °C erhalten.

### Beispiel 3

### 7H-Benzo[e]pyrido[3,4-b]-indol-11-methoxymethyl-10-carbonsäure-isopropylester

a) Analog dem Verfahren im Beispiel 19 von EP-A-54 507wird aus 3H-Benzo[e]indol der 7H-Benzo[e]pryrido[3,4-b]-indol-11-methoxymethyl-10-carbonsäureethylester vom Schmelzpunkt 195 - 197 °C erhalten.
b) Durch Umesterung mit TitanIV-isopropylat erhält man aus dem Ethylester die Titelverbindung vom Schmelzpunkt 163 - 164 °C.

### Beispiel 4

### 7H-Benzo[e]pyrido[3.4-b]-indol-11-methyl-10-carbonsäureisopropylester

a) Analog dem Verfahren im Beispiel 60 der EP-A-54 507 wird aus 3H-Benzo[e]indol der 7H-Benzo[e]pyrido[3,4-b]-indol-11-methyl-10-carbonsäureethylester vom Schmelzpunkt 244 - 246 °C erhalten.
b) Durch Umesterung mit TitanIV-isopropylat erhält man aus dem Ethylester die Titelverbindung vom Schmelzpunkt 171 - 173 °C.

### Beispiel 5

### 7H-Benzo[e]pyrido[3,4-b]-indol-11-ethyl-10-carbonsäureisopropylester

a) Analog dem Verfahren im Beispiel 60 der EP-A-54 507 wird aus 3H-Benzo[e]indol der 7H-Benzo[e]pyrido[3,4,-b]indol-11-ethyl-10-carbonsäureethylester vom Schmelzpunkt 201 - 205 °C erhalten.
b) Durch Umesterung mit TitanIV-isopropylat erhält man aus dem Ethylester die Titelverbindung vom Schmelzpunkt 193 - 196 °C.

### Beispiel 6

### 10-Methyl-2-propyl-oxazolo[4,5-g]-β-carbolin-9-carbonsäureisopropylester

Eine Lösung von 570 mg 6-Hydroxy-4-methyl-β-carbolin-3-carbonsäureisopropylester in 15 ml Ethylengylkoldimethylether wird mit 1 ml n-Butylamin und 5,2 g Mangandioxid versetzt und bei Raumtemperatur über Nacht gerührt. Das Reaktionsgemisch wird über Celite filtriert. Nach dem Einengen der organischen Phase wird der verbleibende Rückstand über Kieselgel mit Essigester chromatographiert. Die gewünschten Fraktionen werden eingeengt und mit Ether ausgerührt.

Man erhält 325 mg 10-Methyl-2-propyl-oxazolo[4,5,-g]-β-carbolin-9-carbonsäureisopropylester vom Schmelzpunkt 223 - 224 °C.

In analoger Weise werden hergestellt:
10-Ethyl-2-isopropyl-oxazolo[4,5-g]-β-carbolin-9-carbonsäureisopropylester
   Schmelzpunkt 205 - 207 °C
10-Ethyl-2-propyl-oxazolo[4,5-g]-β-carbolin-9-carbonsäureisopropylester
   Schmelzpunkt 163 - 165 °C
10-Methyl-2-isopropyl-oxazolo[4,5-g]-β-carbolin-9-carbonsäureisopropylester
   Schmelzpunkt 248 - 249 °C
10-Methoxymethyl-2-ethyl-oxazolo[4,5-g]-β-carbolin-9-carbonsäureisopropylester
   Schmelzpunkt 188 - 189 °C
10-Methoxymethyl-2-methyl-oxazolo[4,5-g]-β-carbolin-9-carbonsäureisopropylester
   Schmelzpunkt 191 - 193 °C
10-Methoxymethyl-2-pentyl-oxazolo[4,5-g]-β-carbolin-9-carbonsäureisopropylester
   Schmelzpunkt 188 - 190 °C
10-Methoxymethyl-2-isopropyl-oxazolo[4,5-g]-β-carbolin-9-carbonsäureisopropylester
   Schmelzpunkt 174 - 176 °C
10-Methoxymethyl-2-phenyl-oxazolo[4,5-g]-β-carbolin-9-carbonsäureisopropylester
   Schmelzpunkt 274 - 276 °C
10-Methoxymethyl-2-propyl-oxazolo[4,5-g]-β-carbolin-9-carbonsäureisopropylester
   Schmelzpunkt 188 - 189 °C
10-Methoxymethyl-2-(2,2-dimethyl-1,3-dioxolan-4-yl)-oxazolo[4,5-g]-β-carbolin-9-carbonsäureisopropylester
   Schmelzpunkt 202 - 203 °C
2-(2,2-Dimethyl-1,3-dioxolan-4-yl)-10-methyl-oxazolo[4,5-g]-β-carbolin-9-carbonsäureisopropylester
   Schmelzpunkt 278 - 280 °C
2-(2,2-Dimethyl-1,3-dioxolan-4-yl)-10-ethyl-oxazolo-[4,5-g]-β-carbolin-9-carbonsäureisopropylester
   Schmelzpunkt 228 - 230 °C

### Beispiel 7

### 2-(1,2-Dihydroxyethyl)-10-methoxymethyl-oxazolo-[4,5-g]-β-carbolin-9-carbonsäureisopropylester

Eine Lösung von 200 mg 2-(2,2-Dimethyl-1,3-dioxolan-4-yl)-10-methoxymethyl-oxazolo-[4,5-g]-β-carbolin-9-carbonsäureisopropylester in 20 ml Methylenchlorid wird tropfenweise bei Raumtemperatur mit 1 ml Trifluoressigsäure versetzt und bei Raumtemperatur unter Schutzgas weitere 4 Stunden gerührt. Die Reaktionslösung wird mit der equimolaren Menge Natriumhydrogencarbonatlösung neutralisiert. Das ausgefallene Festprodukt wird abgesaugt und mit Methylenchlorid gewaschen. Nach dem Trocknen im Vakuum bei 50 °C erhält man 112 mg 2-(1,2-Dihydroxyethyl)-10-methoxymethyl-oxazolo-[4,5-g]-β-carbolin-9-carbonsäureisopropylester vom Schmelzpunkt 168 °C (Zers.)

In analoger Weise werden hergestellt:
2-(1,2-Dihydroxyethyl)-10-methyl-oxazolo-[4,5-g]-β-carbolin-9-carbonsäureisopropylester
   Schmelzpunkt 195 - 196 °C (Zers.)
2-(1,2- Dihydroxyethyl)-10-ethyl-oxazolo-[4,5,-g]-β-carbolin-9-carbonsäureisopropylester
   Schmelzpunkt 184 - 186 °C (Zers).

### Beispiel 8

### 10-Methoxymethyl-2-isopropyl-oxazolo-[4,5-g]-9-(5-methoxymethyl-3-isoxazolyl)-β-carbolin

10-Methoxymethyl-2-isopropyl-oxazolo[4,5-g]6-tosyl-β-carbolin-3-carbaledehydoximhydrochlorid in 6 ml abs. Tetrahydrofuran werden 1,4 ml Natriumhypochlorit lösung bei Raumtemperatur unter Schutzgas zugetropft. Man rührt bis das Oxim verschwunden ist (DC-Kontrolle) 1 Stunde bei Raumtemperatur, dann werden 210 mg Methylpropargylether zugetropft und über Nacht gerührt. Nach Abdestellieren des Lösungsmittels wird in Essigester/Wasser verteilt und die organische Phase getrocknet, filtriert und eingeengt. Der Rückstand wird in 8 ml Methanol gelöst, mit 60 mg Natriummethylat versetzt und 1 Stunde zum Rückfluß erhitzt. Nach Einengen der organischen Phase wird der Rückstand über Kieselgel mit Toluol : Essigester = 1: 1 chromatographiert. Die gewünschten Fraktionen werden eingeengt und aus Essigester kristallisiert. Man erhält 81 mg 10-Methoxymethyl-2-isopropyl-oxazolo[4,5-g]-9-(5-methoxymethyl-3-isoxazolyl)-β-carbolin vom Schmelzpunkt 121 - 122 °C.

Das als Ausgangsmaterial benötigte Carbaldehydoximhydrochlorid wird nach dem in Patent EP 0305 322 beschriebenen Verfahren hergestellt.

Analog werden hergestellt:
10-Methoxymethyl-2-isopropyl-oxazolo[4,5-g]-9-(5-methyl-3-isoxalzolyl)-β-carbolin
   Schmelzpunkt 252 - 255 °C

### Beispiel 9

### 2-Amino-10-ethyl-thiazolo[4.5-g]-β-carbolin-9-carbonsäureisopropylester

297 mg 6-Amino-4-ethyl-β-carbolin-3-carbonsäureisopropylester werden in 5 ml Essigsäure gelöst, mit 152 mg Ammoniumthiocyanat versetzt und 1 Stunde bei Raumtemperatur gerührt. Anschließend wird die Reaktionslösung auf 10 °C abgekühlt und tropfenweise mit 0,05 ml essigsaurer Bromlösung (0,5 ml Br₂ in 9,5 ml Essigsäure) versetzt. Es wird 1 Stunde bei 10 °C nachgerührt und anschließend auf Raumtemperatur erwärmt. Das Reaktionsgemisch wird in Essigester/Wasser aufgenommen und mit 10%iger K₂CO₃-Lösung neuralisiert. Die organische Phase wird abgetrennt, getrocknet und zur Trockene eingeengt. Der Rückstand wird mit Ether verrieben. Man erhält 214 mg der Titelverbindung vom Schmelzpunkt 160 °C (Zers.)

### Beispiel 10

### 2-Amino-10-methoxymethyl-thiazolo[4,5-g-]-β-carbolin-9-carbonsäureisopropylester

Aus 6-Amino-4-methoxymethyl-β-carbolin-3-carbonsäureisopropylester erhält man nach dem im Beispiel 9 beschriebenen Verfahren die Titelverbindung vom Schmelzpunkt 188 -192 °C (Zers.).

### Beispiel 11

### 2-Acetamido-10-ethyl-thiazolo[4,5-g]-β-carbolin-9-carbonsäureisopropylester

100 mg 2-Amino-10-ethyl-thiazolo[4,5-g]-ß-carbolin-9-carbonsäureisopropylester werden in 10 ml Essigsäureanhydrid suspendiert und 15 Minuten auf 80 °C erwärmt. Nach dem Abkühlen wird der ausgefallene Niederschlag abfiltriert und aus Essigester umkristallisiert. Man erhält 51 mg 2-Acetamido-10-ethyl-thiazolo[4,5-g]-β-carbolin-9-carbonsäureisopropylester vom Schmelzpunkt 208 - 210 °C.

### Beispiel 12

### 2-Ethylamino-10-ethyl-thiazolo[4.5-g]-β-carbolin-9-carbonsäureisopropylester

a) 297 mg 6-Amino-4-ethyl-β-carbolin-3-carbonsäureisopropylester und 88 mg Methylisothiocyanat werden in 20 ml Isopropanol 2 Stunden zum Rückfluß erhitzt. Das Lösungsmittel wir im Vakuum abdestelliert und der Rückstand aus Essigester/Ether umkristallisiert. Man erhält 257 mg 4-Ethyl-6-(3-ethyl-thioureido)-β-carbolin-3-carbonsäure-isopropylester vom Schmelzpunkt 234 - 236 °C.
b) Bei Raumtemperatur werden 0,035 ml Brom zu einer Suspension von 180 mg 4-Ethyl-6-(3-ethyl-thioureido)-β-carbolin-3-carbonsäureisopropylester in 20 ml Chloroform getropft und anschließend 3 Stunden zum Rückfluß erhitzt. Die Reaktionslösung wird eingeengt und in Essigester und 20%iger wäßriger K₂CO₃-Lösung aufgenommen. Die organische Phase wird abgetrennt, getrocknet und eingeengt. Der Rückstand wird aus Essigester umkristallisiert. Man erhält 141 mg der Titelverbindung vom Schmelzpunkt 275 - 276 °C.

### Beispiel 13

Analog dem im Beispiel 11 beschriebenen Verfahren werden hergestellt:
2-Methylamino-10-ethyl-thiazolo[4,5-g]-β-carbolin-9-carbonsäureisopropylester
   Schmelzpunkt 200 °C (Zers.)
2-Methylamino-10-methyl-thiazolo[4,5-g]-β-carbolin-9-carbonsäureisopropylester
2-Methylamino-10-methoxymethyl-thiazolo[4,5-g]-β-carbolin-9-carbonsäureisopropylester

### Beispiel 14

### Pyrrolo[4,5-g]-β-carbolin-2,5-dicarbonsäurediethylester

a) 710 mg 6-Amino-β-carbolin-3-carbonsäureethylester werden in 18 ml Wasser bei 4 °C mit 1,2 ml konzentrierter Salzsäure versetzt. Zum dem ausgefallenen Salz wird eine Lösung von 210 mg Natriumnitrit in 20 ml Wasser getropft und weitere 15 Minuten bei 4 °C gerührt. Diese Lösung wird bei 4 °C zu 410 mg Ethyl-2-methylacetacetat und 1 ml 50%ige KOH in 3 ml Ethanol und 6 ml Wasser getropft und weitere 3 Stunden gerührt. Das Reaktionsgemisch wird mit 50 ml Wasser versetzt und mit Essigester extrahiert. Die organischen Phasen werden mit Wasser gewaschen, getrocknet und eingeengt. Man erhält 250 mg 6-(1-Ethoxy-carbonyl-ethylidenhydrazino)-β-carbolin-3-carbonsäureethylester, der ohne weitere Reinigung verarbeitet wird..
b) 750 mg 6-(1-Ethoxycarbonylethylidenhydrazino)-β-carbolin-3-carbonsäureethylester werden mit 1,83 g Polyphosphorsäureethylester in 35 ml abs. Xylol unter Schutzgas 2 Stunden am Rückfluß erhitzt. Nach dem Abkühlen wird das Xylol abdekantiert und der Rückstand in Essigester aufgenommen, über Celite filtriert und eingeengt. Der erhaltene Rückstand wird über Kieselgel mit Ethanol chromatographiert. Die gewünschten Fraktionen werden eingeengt und aus Ethanol umkristallisiert. Man erhält 45 mg der Titelverbindung vom Schmelzpunkt 198 - 201 °C

### Beispiel 15

### 2-Isopropyl-10-methoxymethyl-1H-imidazo[4,5-g-]-β-carbolin-9-carbonsäureisopropylester

627 mg 6-Amino-4-methoxymethyl-β-carbolin-3-carbonsäureisopropylester werden in 10 ml Ethylenglykoldimethylether mit 1,6 g Mangandioxid und 0,98 ml Isobutylamin 16 Stunden bei Raumtemperatur gerührt. Das Reaktionsgemisch wird über Celite filtriert, das Filtrat eingeengt und über Kieselgel mit Methylenchlorid und Ethanol = 10 + 1 chromatographiert. Aus den gewünschten Fraktionen erhält man 499 mg 2-Isopropyl-10-methoxymethyl-1Himidazo[4,5-g]-β-carbolin-9-carbonsäureisopropylester (ölig)

Analog werden hergestellt:
2-Isopropyl-10-methyl-1H-imidazo[4,5-g]-β-carbolin-9-carbonsäureisopropylester
2-Isopropyl-10-ethyl-1H-imidazo[4,5-g]-β-carbolin-9-carbonsäureisopropylester
2-Phenyl-10-methoxymethyl-1H-imidazo[4,5-g]-β-carbolin-9-carbonsäureisopropylester
2-Butyl-10-methoxymethyl-1H-imidazo[4,5-g]-β-carbolin-9-carbonsäureisopropylester
2-(2,2-Dimethyl-1,3-dioxolan-4-yl)-10-ethyl-1H-imidazo[4,5-g]-β-carbolin-9-carbonsäureisopropylester
2-(2,2-Dimethyl-1,3-dioxolan-4-yl)-10-methoxymethyl-1H-imidazo[4,5-g]-β-carbolin-9-carbonsäureisopropylester
2-Ethyl-10-methoxymethyl-1H-imidazo[4,5-g]-β-carbolin-9-carbonsäureisopropylester
10-Methoxymethyl-2-trifluormethyl-1H-imidazo[4,5-g]-β-carbolin-9-carbonsäureisopropylester
10-Methoxymethyl-2-(2-thienyl)-1H-imidazo[4,5-g]-β-carbolin-9-carbonsäureisopropylester
2-(2-Furyl)-10-methoxymethyl-1H-imidazo[4,5-g]-β-carbolin-9-carbonsäureisopropylester
2-(4-Chlorphenyl)-10-methoxymethyl-1H-imidazo[4,5-g]-β-carbolin-9-carbonsäureisopropylester
   Schmelzpunkt 270 °C (Zers.)
2-(2-Chlorphenyl)-10-methoxymethyl-1H-imidazo[4,5-g]-β-carbolin-9-carbonsäureisopropylester
   Schmelzpunkt 205 - 207 °C
2-(4-Methylphenyl)-10-methoxymethyl-1H-imidazo[4,5-g]-β-carbolin-9-carbonsäureisopropylester
   Schmelzpunkt 168 °C (Zers.)
2-(4-Methoxyphenyl)-10-methoxymethyl-1H-imidazo[4,5-g]-β-carbolin-9-carbonsäureisopropylester
   Schmelzpunkt 258 - 260 °C
2-(2-Methoxyphenyl)-10-methoxymethyl-1H-imidazo[4,5-g]-β-carbolin-9-carbonsäureisopropylester
   Schmelzpunkt 225 - 228 °C

### Beispiel 16

Analog dem im Beispiel 7 beschriebenen Verfahren werden hergestellt:
2-(1,2-Dihydroxyethyl)-10-methoxymethyl-1H-imidazo[4,5-g]-β-carbolin-9-carbonsäureisopropylester
   Schmelzpunkt 198 °C
2-(1,2-Dihydroxyethyl)-10-ethyl-1H-imidazo[4,5-g]-β-carbolin-9-carbonsäureisopropylester
   Schmelzpunkt 168 °C

### Beispiel 17

### 10-Ethyl-2-methyl-thiazolo[5,4-g]-β-carbolin-9-carbonsäureisopropylester

a) 2500 mg 6-Amino-4-ethyl-β-carbolin-3-carbonsäureisopropylester werden in 40ml Pyridin gelöst, mit 0,79 ml Acetanhydrid versetzt und 2 Stunden auf 50°C erwärmt. Nach Zugabe von 20ml Wasser wird i.V. eingeengt. Der Rückstand in Essigester gelöst und mit Wasser gewaschen. Die organische Phase wird abgetrennt, getrocknet und zur Trockene eingeengt. Der Rückstand, 3110 mg 6-Acetylamino-4-ethyl-β-carbolin-3-carbonsäureisopropylester, wird ohne weitere Reinigung verarbeitet.
b) 2741 mg 6-Acetylamino-4-ethyl-β-carbolin-3-carbonsäureisopropylester werden in 110 ml Dioxan mit 3915 mg Lawesson's Reagenz unter Rühren auf 70°C erhitzt. Nach dem Abkühlen wird mit 100 ml Essigester versetzt und mit ges.
   Natriumchloridlösung gewaschen. Die organischen Phasen werden getrocknet und i.V. eingeengt. Der Rückstand wird über Kieselgel mit Toluol + Methanol = 8 + 2 chromatographiert. Aus den gewünschten Fraktionen erhält man 1541 mg 4-Ethyl-6-thioacetylamino-β-carbolin-3-carbonsäureisopropylester vom Schmelzpunkt 158-160°C.
c) 450 mg K₃Fe(CN)₆ werden in 1,8 ml Wasser gelöst, mit 1,4 ml 1n NaOH versetzt und auf 4°C abgekühlt. Zu dieser Lösung werden 200 mg 4-Ethyl-6-thioacetylamino-β-carbolin-3-carbonsäureisopropylester in 4 ml Pyridin getropft und weitere 2 Stunden bei dieser Temperatur gerührt. Das Reaktionsgemisch wird in Essigester aufgenommen, mit Wasser gewaschen, getrocknet und eingeengt. Der Rückstand wird über Kieselgel mit Toluol + Ethanol = 95 + 5 chromatographiert.Die gewünschten Fraktionen werden eingeengt und mit Ether verrührt. Man erhält 30 mg der Titelverbindung vom Schmelzpunkt 239-240°C.

## Patentansprüche

1. Verbindungen der Formel I, deren optische Isomere, Tautomere und physiologisch verträglichen Salze 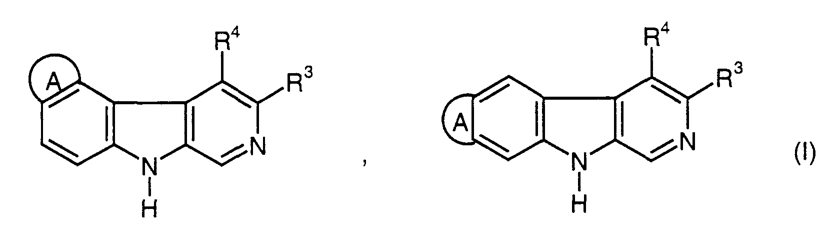 worin
R³ -CO-R^{1,}
R⁴ Wasserstoff, C₁₋₆-Alkyl, C₁₋₄-Alkoxy-C₁₋₂-alkyl,
A einen 5-6 gliedrigen ungesättigten Ring darstellt, in dem 1 - 2 C-Atome durch N, O und/oder S ersetzt sein können und der mit R⁵ und R⁶ substituiert sein kann, und
R⁵ und R⁶ gleich oder verschieden sind und Wasserstoff, C₁₋₆-Alkyl, C₁₋₆-Alkoxy, Hydroxy, das mit leicht abspaltbaren Etherresten, C₁₋₆-Alkanoyl, Benzoyl, zu 1,3-Dioxan, zu 1,3-Dioxolan, zu 2-Phenyl-1,3-dioxan oder zu 2,2-Dimethyl-1,3-dioxolan funktionell abgewandelt sein kann, NR⁷R⁸, COR, C₁₋₆-Alkyl, das gegebenenfalls mit leicht abspaltbaren Etherresten, C₁₋₆-Alkanoyl, Benzoyl, zu 1,3-Dioxan, zu 1,3-Dioxolan, zu 2-Phenyl-1,3-dioxan oder zu 2,2-Dimethyl-1,3-dioxolan funktionell abgewandeltem Hydroxy, C₁₋₄-Alkoxy oder Halogenen substituiert ist, einen C₆₋₁₂-Aryl oder einen 5-6-gliedrigen Hetarylrest, der ein- bis drei N-, O- und/oder S-Atome enthält, bedeuten und der Aryl- und Hetarylrest ein- oder mehrfach mit C₁₋₄-Alkyl, C₁₋₄-Alkoxy, Halogen oder CF₃ substituiert sein kann, oder
R ⁵ und R⁶ gemeinsam eine -(CH₂)ₙ-Gruppe bedeuten und
R¹ und R Hydroxy, C₁₋₆-Alkoxy oder NR¹⁰R¹¹,
n 3 oder 4,
R⁷ und R⁸ jeweils Wasserstoff, C₁₋₆-Alkyl, Acyl oder Phenyl, das ein- oder mehrfach mit C₁₋₄-Alkyl, C₁₋₄-Alkoxy, Halogen oder CF₃ substituiert sein kann,
R¹⁰ und R¹¹ jeweils Wasserstoff, C₁₋₆-Alkyl, C₃₋₇-Cycloalkyl oder einen C₆₋₁₂-Aryl- oder einen 5-6-gliedrigen Hetarylrest, der ein- bis drei N-, O- und/oder S-Atome enthält,
bedeuten und der Aryl- und Hetarylest ein- oder mehrfach mit C₁₋₄-Alkyl, C₁₋₄-Alkoxy, Halogen oder CF₃ substituiert sein kann.

2. Eine Verbindung nach Anspruch 1 worin A ―N=CR⁵-CR⁶=N― bedeutet.

3. Isopropyl-11-ethyl-3-methyl-pyrazino[2,3-g]-β-carbolin-10-carboxylat und Isopropyl-11-ethyl-2-methyl-pyrazino[2,3-g]-β-carbolin-10-carboxylat
Isopropyl-11-methoxymethyl-2,3,4,5-tetrahydroquinoxalino[2,3-g]-β-carbolin-12-carboxylat
Isopropyl-3,11-bismethoxymethyl-pyrazino[2,3-g]-β-carbolin-10-carboxylat
7H-Benzo[e]pyrido[3,4-b]-indol-11-methoxymethyl-10-carbonsäure-isopropylester
10-Methyl-2-propyl-oxazolo-[4,5-g]-β-carbolin-9-carbonsäure-isopropylester
2-(1,2-Dihydroxyethyl)-10-methoxymethyl-oxazolo-[4,5-g]-β-carbolin-9-carbonsäure-isopropylester
10-Methoxymethyl-2-isopropyl-oxazolo-[4,5-g]-9-(5-methoxymethyl-3-isoxazolyl)-β-carbolin
2-Amino-10-ethyl-thiazolo-[4,5-g]-β-carbolin-9-carbonsäure-isopropylester
2-Amino-10-methoxymethyl-thiazolo[4,5-g-]-β-carbolin-9-carbonsäure-isopropylester
Pyrrolo[4,5-g]-β-carbolin-2,5-dicarbonsäurediethylester
2-Isopropyl-10-methoxymethyl-1H-imidazo[4,5-g-]-β-carbolin-9-carbonsäure-isopropylester
10-Ethyl-2-methyl-thiazolo[5,4-g]-β-carbolin-9-carbonsäure-isopropylester

4. Arzneimittel enthaltend eine Verbindung nach Anspruch 1 - 3 und einen oder mehrere pharmazeutisch übliche Träger- oder Hilfsstoffe.

5. Verwendung der Verbindungen nach Anspruch 1 - 3 zur Herstellung von Arzneimitteln zur Behandlung geriatrischer Beschwerden und cognitiver Defizite.

6. Verfahren zur Herstellung von Verbindungen nach Anspruch 1, **dadurch gekennzeichnet, daß** man
a) eine Verbindung der Formel II 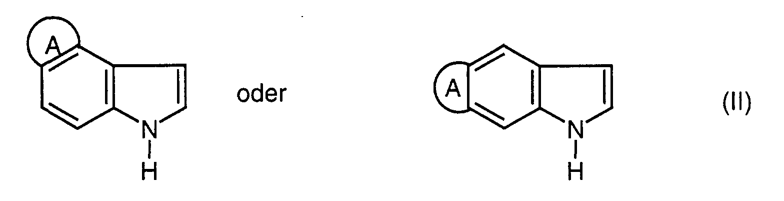 mit einem 2-Azadien der Formel III 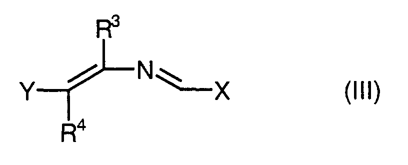 worin R³, R⁴ und A die obige Bedeutung haben, und X und Y Fluchtgruppen darstellen, in Gegenwart von Säuren umsetzt oder
b) eine Verbindung der Formel IV 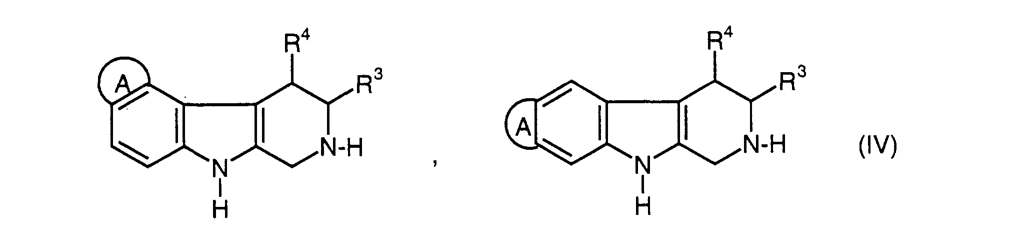 worin R³, R⁴ und A die obige Bedeutung haben, aromatisiert oder
c) eine Verbindung der Formel V 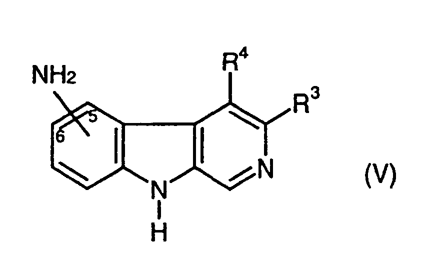 worin R³ und R⁴ die obige Bedeutung haben, mit einem α, β-ungesättigtem Aldehyd umsetzt zu einem ankondensierten Pyridin oder mit einem primären Amin H₂N-CH₂-R⁵ umsetzt zu einem ankondensierten Imidazol oder die mit Nitriten erhaltenen Diazoniumsalze mit Acetessigsäurederivaten umsetzt zu einem Ethylidenhydrazinderivat und dieses zum Pyrrol cyclisiert oder mit Thiocyanat- oder Isothiocyanatderivaten umsetzt zu einem ankondensierten Thiazol oder
d) eine Verbindung der Formel VI 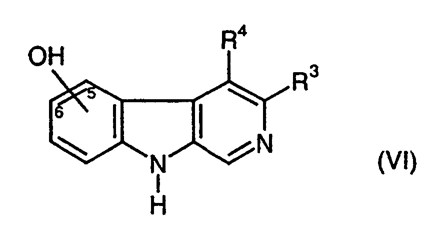 worin R³ und R⁴ die obige Bedeutung haben, mit einem primären Amin H₂N-CH₂-R⁵ zu einem ankondensierten Oxazol umsetzt oder mit einem vicinalen primären Diamin 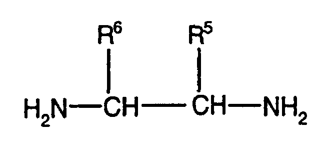 zu einem ankondensierten Pyrazin umsetzt oder
e) ein Nitriloxid der Formel VII 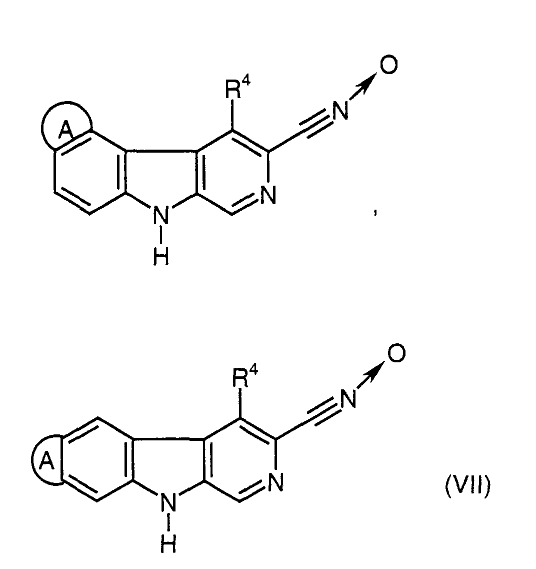 mit ≡―R² zu einem Isoxazolderivat cyclisiert,
und gewünschtenfalls anschließend eine Estergruppe verseift oder umestert, eine Carboxylgruppe verestert, eine Aminogruppe alkyliert oder acyliert, eine funktionell abgewandelte Hydroxygruppe freisetzt, die Isomeren trennt oder die physiologisch verträglichen Salze bildet.

## Claims

1. Compounds of the formula I, and isomers, tautomers or physiologically tolerated salts thereof 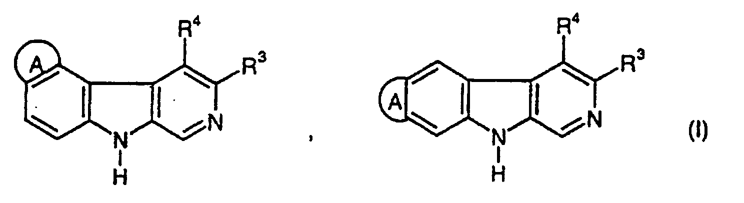 in which
R³ is -CO-R¹,
R⁴ is hydrogen, C₁₋₆ alkyl, or C₁₋₄ alkoxy-C₁₋₂ alkyl,
A is a 5-6-membered unsaturated ring, in which 1-2 C atoms may be replaced by N, O and/or S and which may be substituted by R⁵ and R⁶, and
R⁵ and R⁶ are the same or different and are hydrogen, C₁₋₆ alkyl, C₁₋₆ alkoxy, hydroxyl which may be functionally modified with easily eliminated ether residues, C₁₋₆ alkanoyl, benzoyl, to 1,3-dioxane, to 1,3-dioxolane, to 2-phenyl-1, 3-dioxane or to 2,2-dimethyl-1, 3-dioxolane, or are NR⁷R⁸, COR, C₁₋₆ alkyl optionally substituted by hydroxyl functionally modified C₁₋₄ alkoxy or halogens, a C₆₋₁₂ aryl or a 5-6-membered hetaryl radical which contains one to three N, O and/or S atoms, and the aryl and hetaryl radical may be substituted with easily eliminated ether residues, C₁₋₆ alkanoyl, benzoyl, to 1,3-dioxane, to 1,3-dioxolane, to 2-phenyl-1, 3-dioxane or to 2,2-dimethyl-1, 3-dioxolane, or one or more times by C₁₋₄ alkyl, C₁₋₄ alkoxy, halogen or CF₃, or
R⁵ and R⁶ together are a -(CH₂)ₙ - group and
R¹ and R are hydroxyl, C₁₋₆ alkoxy or NR¹⁰ R¹¹,
n is 3 or 4,
R⁷ and R⁸ are each hydrogen, C₁₋₆ alkyl, acyl or phenyl which may be substituted one or more times by C₁₋₄ alkyl, C₁₋₄ alkoxy, halogen or CF₃,
R¹⁰ and R¹¹ are each hydrogen, C₁₋₆ alkyl, C₃₋₇ cycloalkyl or a C₆₋₁₂ aryl radical or a 5-6-membered hetaryl radical which contains one to three N, O and/or S atoms, and the aryl and hetaryl radicals may be substituted one or more times by C₁₋₄ alkyl, C₁₋₄ alkoxy, halogen or CF₃.

2. A compound according to Claim 1, in which A is -N=CR⁵-CR⁶=N-.

3. Isopropyl 11-ethyl-3-methylpyrazino[2,3g]-β-carboline-10-carboxylate and
isopropyl 11-ethyl-2-methylpyrazino[2,3-g]-β-carboline-10-carboxylate
isopropyl 11-methoxymethyl-2,3,4,5-tetrahydroquinoxalino[2,3g]-β-carboline-12-carboxylate,
isopropyl 3,11-bismethoxymethylpyrazino[2,3-g]-β-carboline-10-carboxylate,
11-methoxymethyl-7H-benzo[e]pyrido[3,4-b]-indole-10-carboxylic acid isopropyl ester,
10-methyl-2-propyloxazolo[4,5-g]-β-carboline-9-carboxylic acid isopropyl ester,
2-(1,2-dihydroxyethyl)-10-methoxymethyloxazolo-[4,5-g]-β-carboline-9-carboxylic acid isopropyl ester,
10-methoxymethyl-2-isopropyloxazolo-[4,5-g]-9-(5-methoxymethyl-3-isoxazolyl)-β-carboline,
2-amino-10-ethylthiazolo[4,5-g]-β-carboline-9-carboxylic acid isopropyl ester,
2-amino-10-methoxymethyl-thiazolo[4,5-g]-β-carboline-9-carboxylic acid isopropyl ester,
pyrrolo[4,5-g]-β-carboline-2,5-dicarboxylic acid diethyl ester,
2-isopropyl-10-methoxymethyl-1H-imidazo[4,5-g]-β-carboline-9-carboxylic acid isopropyl ester
10-ethyl-2-methyl-thiazolo[5,4-g]-β-carboline-9-carboxylic acid isopropyl ester.

4. Pharmaceutical composition which comprises a compound according to Claim 1-3 and one or more pharmaceutically acceptable carriers and/or excipients.

5. Use of compounds according to Claim 1-3 for producing pharmaceutical compositions for the treatment of geriatric symptoms and cognitive deficits.

6. Process for the preparation of compounds according to Claim 1, **characterized in that**:
a) a compound of formula II 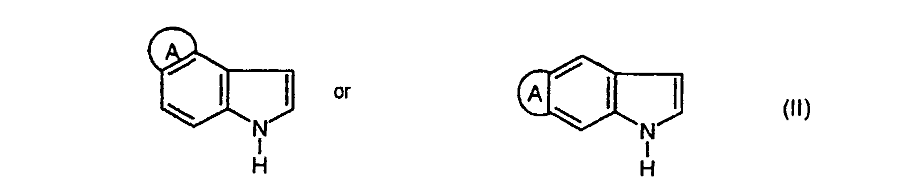 is reacted with a 2-azadiene of formula III 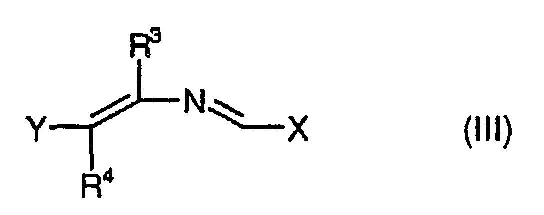 in which R³, R⁴ and A have the above meaning, and X and Y are leaving groups, in the presence of acids
or
b) a compound of formula IV 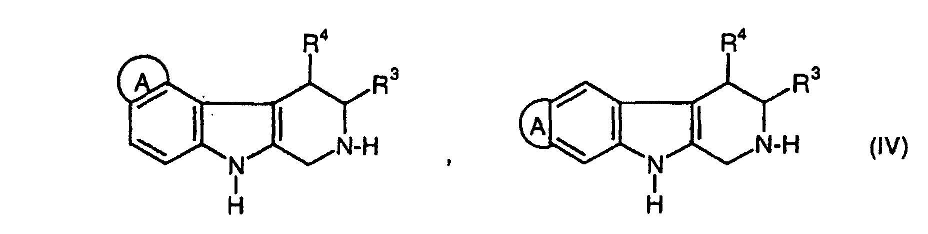 in which R³, R⁴ and A have the above meaning is aromatized, or
c) a compound of formula V 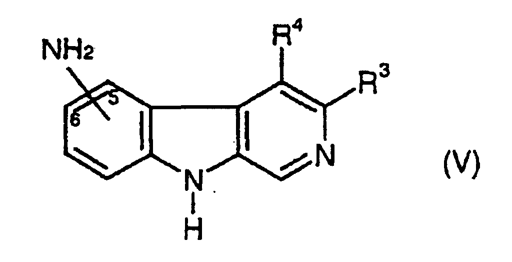 in which R³ and R⁴ have the above meaning, is reacted with an α, β-unsaturated aldehyde to give a fused pyridine or with a primary amine H₂N-CH₂-R⁵ to give a fused imidazole, or the diazonium salts obtained with nitrites are reacted with acetoacetic acid derivatives to give an ethylidenehydrazine derivative and the latter is cyclized to the pyrrole, or reacted with thiocyanate or isothiocyanate derivatives to give a fused thiazole or
d) a compound of formula VI 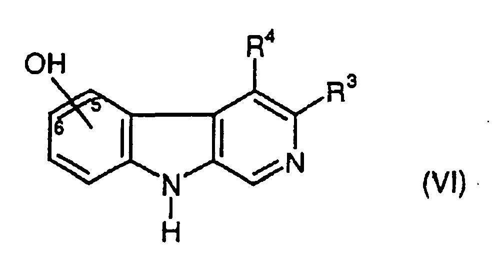 in which R³ and R⁴ have the above meaning, is reacted with a primary amine H₂N-CH₂-R⁵ to give a fused oxazole or with a vicinal primary diamine 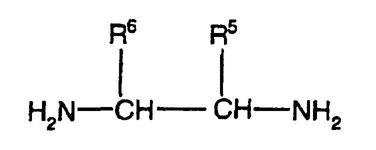 to, are a fused pyrazine or
e) a nitrile oxide of formula VII 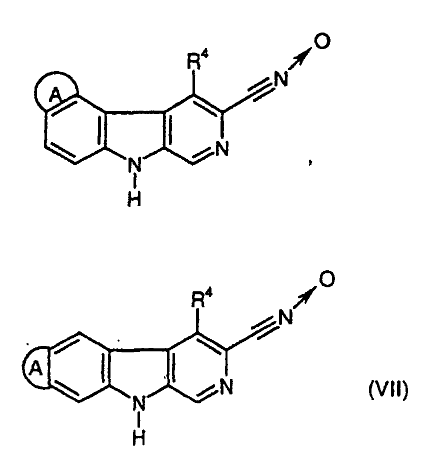 is cyclized with =-R² to give an isoxazole derivative,
and if desired then an ester group is hydrolyzed or transesterified, a carboxyl group is esterified, an amino group is alkylated or acylated, a functionally modified hydroxyl group is liberated, the isomers are separated or physiologically tolerated salts are formed.

## Revendications

1. Composés de formule I, leurs isomères optiques, tautomères et sels physiologiquement acceptables 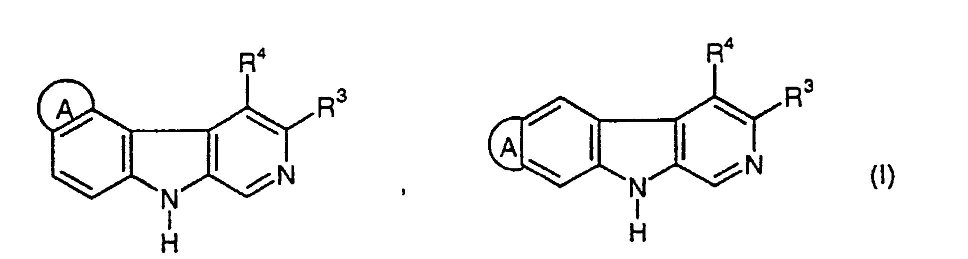 formules dans lesquelles
R³ représente -CO-R¹,
R⁴ représente un atome d'hydrogène ou un groupe alkyle en C₁₋₆, alcoxy(C₁₋₄)-alkyle(C₁₋₂),
A représente un cycle insaturé à 5-6 chaînons, dans lequel 1-2 atomes de carbone peuvent être remplacés par N, O et/ou S et qui peut être substitué par R⁵ et R⁶, et
R⁵ et R⁶ sont identiques ou différents et représentent un atome d'hydrogène ou un groupe alkyle en C₁₋₆, alcoxy en C₁₋₆, hydroxy qui peut être converti fonctionnellement, avec des radicaux éther aisément séparables, alcanoyle en C₁₋₆, benzoyle, en 1,3-dioxanne, en 1,3-dioxolanne, en 2-phényl-1,3-dioxanne ou en 2,2-diméthyl-1,3-dioxolanne, un groupe NR⁷R⁸, COR, alkyle en C₁₋₆ qui est éventuellement substitué par un atome d'halogène ou par un groupe alcoxy en C₁₋₄ ou hydroxy converti fonctionnellement, avec des radicaux éther aisément séparables, alcanoyle en C₁₋₆, benzoyle, en 1,3-dioxanne, en 1,3-dioxolanne, en 2-phényl-1,3-dioxanne ou en 2,2-diméthyl-1,3-dioxolanne, un groupe aryle en C₆₋₁₂ ou un radical hétéroaryle à 5-6 chaînons, qui comporte un à trois atomes de N, O et/ou S, et les radicaux aryle et hétéroaryle peuvent être une ou plusieurs fois substitués par un ou plusieurs atomes d'halogène ou groupes alkyle en C₁₋₄, alcoxy en C₁₋₄ ou CF₃, ou
R⁵ et R⁶ représentent ensemble un groupe -(CH₂)ₙ- et
R¹ et R représentent un groupe hydroxy, alcoxy en C₁₋₆ ou NR¹⁰R¹¹,
n est 3 ou 4,
R⁷ et R⁸ représentent chacun un atome d'hydrogène, un groupe alkyle en C₁₋₆, acyle ou phényle, qui peut être une ou plusieurs fois substitué par un ou des atomes d'halogène ou groupes alkyle en C₁₋₄, alcoxy en C₁₋₄ ou CF₃,
R¹⁰ et R¹¹ représentent chacun un atome d'hydrogène, un groupe alkyle en C₁₋₆, cycloalkyle en C₃₋₇ ou un radical aryle en C₆₋₁₂ ou hétéroaryle à 5 ou 6 chaînons, qui comporte un à trois atomes de N₉ O et/ou S, et les radicaux aryle et hétéroaryle peuvent être une ou plusieurs fois substitués par un ou des atomes d'halogène ou groupes alkyle en C₁-C₄, alcoxy en C₁-C₄ ou CF₃.

2. Composé selon la revendication 1, dans lequel A représente un groupe -N=CR⁵-CR⁶=N-.

3. 11-éthyl-3-méthyl-pyrazino[2,3-g]-β-carboline-10-carboxylate d'isopropyle et
11-éthyl-2-méthyl-pyrazino[2,3-g]-β-carboline-10-carboxylate d'isopropyle
11-méthoxyméthyl-2,3,4,5-tétrahydroquinoxalino[2,3-g]-β-carboline-12-carboxylate d'isopropyle
3,11-bis-méthoxyméthyl-pyrazino[2,3-g]-β-carboline-10-carboxylate d'isopropyle
7H-benzo[e]pyrido[3,4-b]-indole-11-méthoxyméthyl-10-carboxylate d'isopropyle
10-méthyl-2-propyl-oxazolo-[4,5-g]-β-carboline-9-carboxylate d'isopropyle
2-(1,2-dihydroxyéthyl)-10-méthoxyméthyl-oxazoloéthyl-[4,5-g]-β-carboline-9-carboxylate d'isopropyle.
10-méthoxyméthyl-2-isopropyl-oxazolo-[4,5-g]-9-(5-méthoxyméthyl)-3-isoxazolyl)-β-carboline
2-amino-10-éthyl-thiazolo-[4,5-g]-β-carboline-9-carboxylate d'isopropyle
2-amino-10-méthoxyméthyl-thiazolo[4,5-g]-β-carboline-9-carboxylate d'isopropyle
pyrrolo[4,5-g]-β-carboline-2,5-dicarboxylate de diéthyle
2-isopropyl-10-méthoxyméthyl-1H-imidazo[4,5-g]-β-carboline-9-carboxylate d'isopropyle
10-éthyl-2-méthyl-thiazolo[5,4-g]-β-carboline-9-carboxylate d'isopropyle.

4. Médicaments contenant un composé selon l'une quelconque des revendications 1 à 3 et un ou plusieurs adjuvants ou véhicules usuels en pharmacie.

5. Utilisation des composés selon l'une quelconque des revendications 1 à 3, pour la fabrication de médicaments destinés au traitement de troubles gériatriques et de déficiences cognitives.

6. Procédé pour la préparation de composés selon la revendication 1, **caractérisé en ce que**
a) on fait réagir un composé de formule II 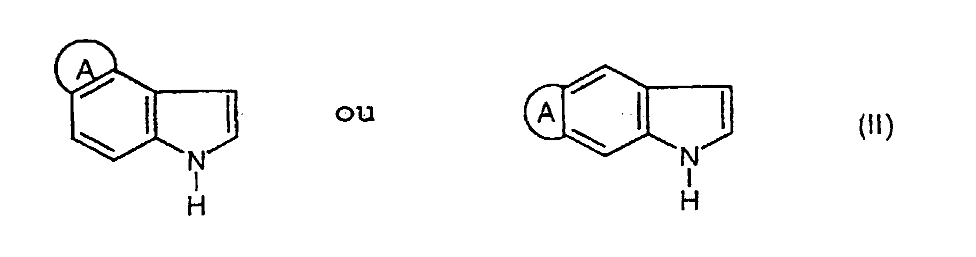 avec un 2-azadiène de formule III 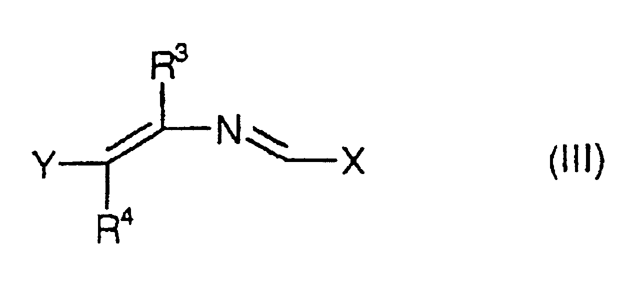 formules dans lesquelles R³, R⁴ et A ont les significations données plus haut, et X et Y représentent des groupes partants, en présence d'acides ou
b) on soumet à une aromatisation un composé de formule IV 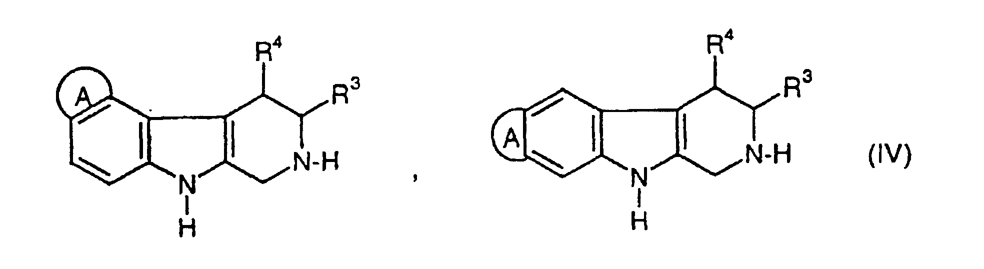 formules dans lesquelles R³, R⁴ et A ont les significations données plus haut, ou
c) on fait réagir un composé de formule V 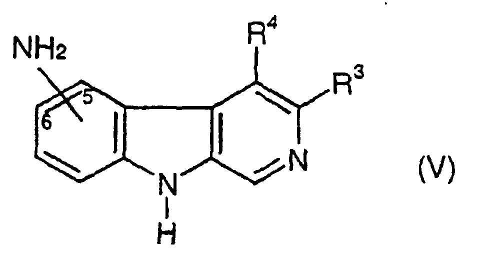 dans laquelle R³ et R⁴ ont les significations données plus haut, avec un aldéhyde α,β-insaturé, pour aboutir à une pyridine condensée, ou avec une amine primaire H₂N-CH₂-R⁵ pour aboutir à un imidazole condensé, ou les sels de diazonium, obtenus avec des nitriles, avec des dérivés d'acide acétoacétique pour aboutir à un dérivé éthylidènehydrazine, et on soumet ce dernier à une cyclisation en le pyrrole ou on le fait réagir avec des dérivés de thiocyanate ou d'isothiocyanate pour aboutir à un thiazole condensé ou
d) on fait réagir un composé de formule VI 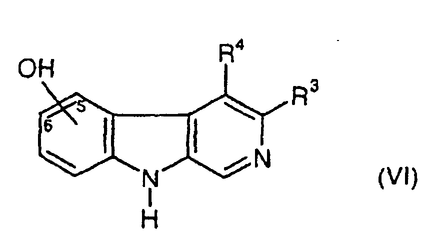 dans laquelle R³ et R⁴ ont les significations données plus haut, avec une amine primaire H₂N-CH₂-R⁵, pour aboutir à un oxazole condensé ou avec une diamine primaire vicinale 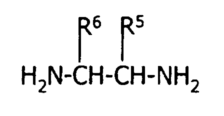 pour aboutir à une pyrazine condensée ou
e) on soumet à une cyclisation un oxyde de nitrile de formule VII 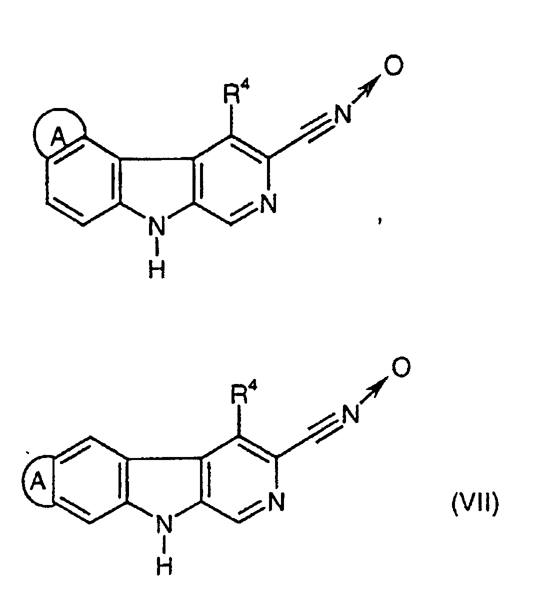 avec ≡―R² pour aboutir à un dérivé isoxazole,
et, si on le désire, ensuite on saponifie ou transestérifie un groupe ester, on estérifie un groupe carboxy, on soumet un groupe amino à une alkylation ou une acylation, on libère un groupe hydroxy converti fonctionnellement, on sépare les isomères ou on forme les sels physiologiquement acceptables.
